(19) Europäisches Patentamt

European Patent Office

Office européen des brevets

(11) **EP 1 714 985 A1**

(12) **EUROPEAN PATENT APPLICATION**
published in accordance with Art. 158(3) EPC

(43) Date of publication:
**25.10.2006 Bulletin 2006/43**

(21) Application number: **04807379.5**

(22) Date of filing: **20.12.2004**

(51) Int Cl.:
*C08F 2/20* (2006.01)          *C08F 2/38* (2006.01)

(86) International application number:
**PCT/JP2004/019022**

(87) International publication number:
**WO 2005/063825 (14.07.2005 Gazette 2005/28)**

(84) Designated Contracting States:
**BE DE FR**

(30) Priority: **25.12.2003 JP 2003430675**

(71) Applicant: **SUMITOMO SEIKA CHEMICALS CO., LTD.**
**Kako-gun, Hyogo 675-0145 (JP)**

(72) Inventors:
• **UDA, Shinichi**
  **Shiga 525-0025 (JP)**

• **KAWAKITA, Tomoki c/o Sumitomo Seika Chemicals**
  **Hyogo 672-8076 (JP)**
• **NAWATA, Yasuhiro Sumimoto Seika Chem. Co Ltd.**
  **Hyogo 672-8076 (JP)**

(74) Representative: **Vossius & Partner**
  **Siebertstrasse 4**
  **81675 München (DE)**

(54) **METHOD FOR PRODUCING WATER-ABSORBING RESIN**

(57)     A process for preparing a water-absorbent resin comprising carrying out a reversed phase suspension polymerization in multi-steps of at least two steps when the water-absorbent resin is prepared by subjecting a water-soluble ethylenically unsaturated monomer to the reversed phase suspension polymerization, said process for preparing a water-absorbent resin being **characterized by** adding a phosphorus-containing compound to at least one step in the second and subsequent steps, to carry out the polymerization reaction. The water-absorbent resin can be suitably used in hygienic materials such as disposable diaper, incontinence pad and sanitary napkin, especially in disposable diaper.

[Figure 1]

**Description**

TECHNICAL FIELD

The present invention relates to a process for preparing a water-absorbent resin. More specifically, the present invention relates to a process for preparing a water-absorbent resin which can be suitably used in hygienic materials such as disposable diaper, incontinence pad and sanitary napkin, especially in disposable diaper.

BACKGROUND ART

Conventionally, water-absorbent resins have been widely used in hygienic materials such as disposable diaper and sanitary napkin, and industrial materials such as water blocking materials for cables. As water-absorbent resins, there have been known, for example, hydrolysates of starch-acrylonitrile graftcopolymers, neutralized products of starch-acrylate graftcopolymers, saponified products of vinyl acetate-acrylic ester copolymers, partially neutralized products of polyacrylic acid, and the like.

In recent years, an absorbent body in a hygienic material such as disposable diaper or sanitary napkin tends to be made thinner from the viewpoint of a comfortable fit upon use. When the absorbent body is thinned, the ratio of a water-absorbent resin in the absorbent body is increased, so that gel blocking of the water-absorbent resins with each other is likely to take place when a body fluid or the like is absorbed. Therefore, in order to suppress the gel blocking of the water-absorbent resins with each other, it is required that the water-absorbent resins have a large amount of water absorption under pressure. However, in order to make the amount of water absorption large under pressure, it is generally necessary to increase a crosslinking density of the water-absorbent resin, whereby consequently having a disadvantage that the water-retaining capacity of the water-absorbent resin is lowered. On the other hand, a water-absorbent resin having a high water-absorption rate is desired in order to prevent leakage of a body fluid or the like. In addition, in order to maintain the comfortable fit of the hygienic material upon a long-term use, a water-absorbent resin having a smaller amount of water-soluble substance has been desired.

As processes for preparing a water-absorbent resin used in a hygienic material, a process comprising carrying out reversed phase suspension polymerization using specified amounts of specified polymeric protective colloid and surfactant (see Patent Publication 1); a process comprising carrying out reversed phase suspension polymerization in multi-steps of at least two steps (see Patent Publication 2); a process comprising carrying out reversed phase suspension polymerization in the presence of a $\beta$-1,3-glucan to give a water-absorbent resin, and adding a crosslinking agent to the water-absorbent resin to carry out a crosslinking reaction (see Patent Publication 3); a process comprising carrying out reversed phase suspension polymerization using a specified amount of a persulfuric acid salt as a polymerization initiator (see Patent Publication 4); a process comprising carrying out aqueous solution polymerization in the presence of phosphorous acid and/or a salt thereof, to give a precursor of a water-absorbent resin, and thereafter mixing the precursor of a water-absorbent resin with a surface crosslinking agent while heating (see Patent Publication 5); and the like have been known. However, the water-absorbent resins obtained by these processes do not sufficiently satisfy required properties such as not only a large water-retaining capacity and a large amount of water absorption under pressure, but also a high water-absorption rate or a small amount of water-soluble substance. There are further rooms for improvement.

Patent Publication 1: Japanese Patent Laid-Open No. Hei 6-345819
Patent Publication 2: Japanese Patent Laid-Open No. Hei 3-227301
Patent Publication 3: Japanese Patent Laid-Open No. Hei 8-120013
Patent Publication 4: Japanese Patent Laid-Open No. Hei 6-287233
Patent Publication 5: Japanese Patent Laid-Open No. Hei 9-124710

DISCLOSURE OF INVENTION

PROBLEMS TO BE SOLVED BY THE INVENTION

An object of the present invention is to provide a process for preparing a water-absorbent resin which can be suitably used in a hygienic material, the water-absorbent resin having a larger amount of water retention, a larger amount of water absorption under pressure, a higher water absorption rate, and a smaller amount of water-soluble substance.

MEANS TO SOLVE THE PROBLEMS

Specifically, the present invention relates to a process for preparing a water-absorbent resin comprising carrying out a reversed phase suspension polymerization in multi-steps of at least two steps when the water-absorbent resin is prepared by subjecting a water-soluble ethylenically unsaturated monomer to the reversed phase suspension polymerization, said process for preparing a water-absorbent resin being characterized by adding a phosphorus-containing compound to at least one step in the second and subsequent steps, to carry out the polymerization reaction.

EFFECTS OF THE INVENTION

According to the process of the present invention, a water-absorbent resin having a larger amount of water retention, a larger amount of water absorption under pressure, a higher water absorption rate, and a smaller amount of water-soluble substance can be obtained.

BRIEF DESCRIPTION OF THE DRAWINGS

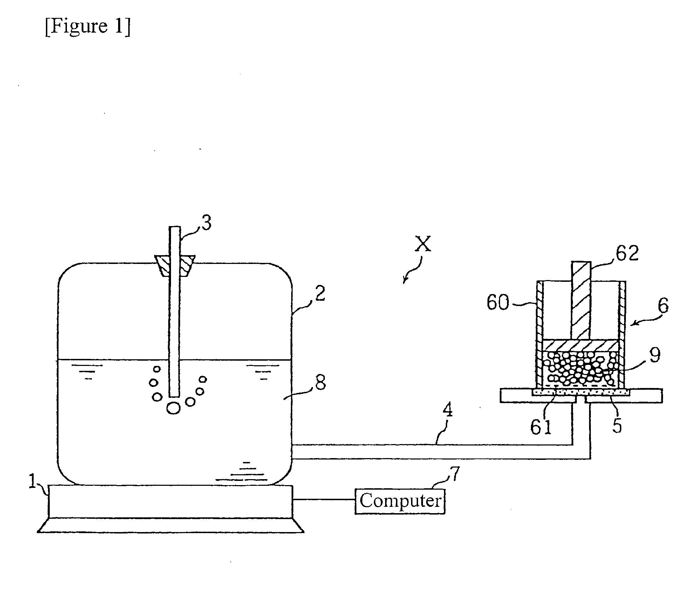

[Figure 1] Figure 1 is a schematic explanatory view of a measuring apparatus X used in the determination of the amount of water absorption of physiological saline under pressure.

EXPLANATION OF NUMERICAL SYMBOLS

X    measuring apparatus
1    balance
2    bottle
3    air aspiration tube
4    lead tube
5    glass filter
6    measuring section
7    computer
8    physiological saline
9    water-absorbent resin
60    cylinder
61    nylon mesh
62    weight

BEST MODE FOR CARRYING OUT THE INVENTION

In the process for preparing a water-absorbent resin of the present invention, first, a first-step reversed phase suspension polymerization in a water-in-oil system is carried out by mixing together an aqueous solution of a water-soluble ethylenically unsaturated monomer, a surfactant and/or a polymeric protective colloid, a water-soluble radical polymerization initiator, and a hydrocarbon-based solvent, and heating the mixture with stirring.

The water-soluble ethylenically unsaturated monomer includes, for example, (meth)acrylic acid ["(meth)acryl-" means "acryl-" or "methacryl-;" hereinafter referred to the same], 2-(meth)acrylamide-2-methylpropanesulfonic acid and an alkali metal salt thereof; nonionic unsaturated monomers such as (meth)acrylamide, N,N-dimethylacrylamide, 2-hydroxyethyl (meth)acrylate, and N-methylol (meth)acrylamide; amino group-containing unsaturated monomers such as diethylaminoethyl (meth)acrylate and diethylaminopropyl (meth)acrylate, or a quaternary salt thereof; and the like. These can be used alone or in admixture of at least two kinds. The alkali metal in the alkali metal salts includes lithium, sodium, potassium, and the like.

Among the water-soluble ethylenically unsaturated monomers, preferred ones include (meth)acrylic acid or an alkali metal salt thereof, acrylamide, methacrylamide and N,N-dimethylacrylamide, from the viewpoint of being industrially easily available. Even more preferred ones include (meth)acrylic acid or an alkali metal salt thereof, from the viewpoint of economical advantage.

The water-soluble ethylenically unsaturated monomer can be usually used in the form of an aqueous solution. It is preferable that the concentration of the water-soluble ethylenically unsaturated monomers in the aqueous solution of the water-soluble ethylenically unsaturated monomers is from 15% by weight to a saturated concentration.

In the aqueous solution of the water-soluble ethylenically unsaturated monomer, when the water-soluble ethylenic monomer to be used has an acid group, the acid group may be neutralized with an alkali metal. It is preferable that the degree of neutralization by the above-mentioned alkali metal is from 10 to 100% by mol of the acid group of the water-soluble ethylenically unsaturated monomer before the neutralization, from the viewpoint of increasing osmotic pressure and increasing water absorption rate of the resulting water-absorbent resin, and not causing any disadvantages in safety or the like due to the presence of an excess alkali metal. The alkali metal includes lithium, sodium, potassium, and the like. Among them, sodium and potassium are preferable, and sodium is more preferable.

The surfactant includes, for example, nonionic surfactants such as sorbitan fatty acid esters, polyglycerol fatty acid esters, sucrose fatty acid esters, sorbitol fatty acid esters, and polyoxyethylene alkylphenyl ethers; anionic surfactants such as fatty acid salts, alkylbenzenesulfonic acid salts, alkyl methyl tauric acid salts, polyoxyethylene alkylphenyl ether sulfate esters, and polyoxyethylene alkyl ether sulfonic acid salts; and the like. Among them, sorbitan fatty acid esters, polyglycerol fatty acid esters, and sucrose fatty acid esters are preferable.

The polymeric protective colloid includes, for example, ethyl cellulose, ethyl hydroxyethyl cellulose, polyethylene oxide, maleic anhydride-modified polyethylene, maleic anhydride-modified polybutadiene, maleic anhydride-modified EPDM (ethylene-propylene-diene terpolymer), and the like.

The amount of the surfactant and/or the polymeric protective colloid is preferably from 0.1 to 5 parts by weight, and more preferably from 0.2 to 3 parts by weight, based on 100 parts by weight of the aqueous solution of the water-soluble ethylenically unsaturated monomer.

The water-soluble radical polymerization initiator includes, for example, persulfates such as potassium persulfate, ammonium persulfate, and sodium persulfate; organic peroxides such as tert-butyl hydroperoxide, cumene hydroperoxide, and benzoyl peroxide; hydrogen peroxide; azo compounds such as 2,2'-azobis(2-amidinopropane)dihydrochloride; and the like. Among them, potassium persulfate, ammonium persulfate, sodium persulfate, benzoyl peroxide, and 2,2'-azobis(2-amidinopropane)dihydrochloride are preferable, from the viewpoint of being easily available and excellent in storage stability. The water-soluble radical polymerization initiator can be used as a redox-system polymerization initiator together with a sulfite or the like.

It is preferable that the amount of the water-soluble radical polymerization initiator is usually from 0.00005 to 0.01 mol, per 1 mol of the water-soluble ethylenically unsaturated monomer, from the viewpoint of shortening the time period for the polymerization reaction and preventing abrupt polymerization reaction.

The hydrocarbon-based solvent includes, for example, aliphatic hydrocarbons such as n-hexane, and n-heptane; alicyclic hydrocarbons such as cyclopentane, methylcyclopentane, cyclohexane, and methylcyclohexane; aromatic hydrocarbons such as benzene, toluene, and xylene; and the like. Among them, n-hexane, n-heptane, and cyclohexane are preferable, from the viewpoint of being industrially easily available, stable in quality and inexpensive.

Usually, the amount of the hydrocarbon-based solvent is preferably from 50 to 600 parts by weight, and more preferably from 80 to 550 parts by weight, based on 100 parts by weight of the water-soluble ethylenically unsaturated monomer, from the viewpoint of removing heat of polymerization and being likely to easily control the polymerization temperature.

In the present invention, it is preferable that the above-mentioned polymerization reaction is carried out in the presence of a crosslinking agent. The crosslinking agent includes, for example, diols, triols and polyols such as (poly) ethylene glycol ["(poly)" means both cases where the prefix "poly" is included and where the prefix is not included; hereinafter referred to the same], (poly)propylene glycol, 1,4-butanediol, trimethylolpropane, polyoxyethylene glycol, polyoxypropylene glycol, and (poly)glycerol; unsaturated polyesters obtained by reacting the above-mentioned diol, triol, or polyol with an unsaturated acid such as (meth)acrylic acid, maleic acid or fumaric acid; bisacrylamides such as N,N-methylenebisacrylamide; di- or tri(meth)acrylate esters obtained by reacting a polyepoxide with (meth)acrylic acid; carbamyl ester of di(meth)acrylic acid obtained by reacting a polyisocyanate such as tolylene diisocyanate or hexamethylene diisocyanate with hydroxyethyl (meth)acrylate; compounds each having at least two polymerizable unsaturated groups, such as allylated starch, allylated cellulose, diallyl phthalate, N,N',N''-triallyl isocyanurate, and divinylbenzene; diglycidyl ether compounds such as (poly)ethylene glycol diglycidyl ether, (poly)propylene glycol diglycidyl ether, and (poly)glycerol diglycidyl ether; epihalohydrin compounds such as epichlorohydrin, epibromohydrin, and $\alpha$-methylepichlorohydrin; compounds each having at least two reactive functional groups, such as isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; and the like. These can be used alone, or in admixture of at least two kinds.

The amount of the crosslinking agent is preferably from 0.000001 to 0.001 mol per 1 mol of the water-soluble ethylenically unsaturated monomer in order to suppress the water solubility of the resulting polymer by an appropriate crosslinking of the polymer, thereby showing a satisfactory water absorbency.

The reaction temperature of the polymerization reaction differs depending upon the radical polymerization initiator used. The reaction temperature is preferably from 20° to 110°C and more preferably from 40° to 80°C, from the viewpoint of rapidly progressing the reaction and shortening the polymerization time, thereby increasing productivity, and easily removing heat of polymerization, thereby smoothly carrying out the reaction. The reaction time is usually from 0.5 to 4 hours.

Thus, the first-step reversed phase suspension polymerization is carried out.

Next, the reaction mixture obtained by the first-step reversed phase suspension polymerization is subjected to a second- or subsequent-step reversed phase suspension polymerization. In the present invention, the reversed phase suspension polymerization is carried out in multi-steps of at least two steps, and it is preferable that the number of steps is 2 or 3 steps from the viewpoint of increasing productivity.

The greatest feature of the present invention resides in that a reversed phase suspension polymerization is carried out in the presence of a phosphorus-containing compound in at least one of the steps of the polymerization in the second or subsequent step. In the present invention, it is preferable that the reversed phase suspension polymerization is carried out in the presence of a phosphorus-containing compound only in at least one step of the second or subsequent steps, without the presence of the phosphorus-containing compound in the first step. The process for carrying out a reversed phase suspension polymerization in the second or subsequent steps in the presence of a phosphorus-containing compound is not particularly limited. One example of the process for carrying out a second- or subsequent-step reversed phase suspension polymerization includes a process comprising adding an aqueous solution of a water-soluble ethylenically unsaturated monomer to the reaction mixture obtained in the first-step polymerization reaction with mixing, and carrying out a second- or subsequent-step reversed phase suspension polymerization in the same manner as in the first step.

The phosphorus-containing compound may be added in a given amount to the aqueous solution of a water-soluble ethylenically unsaturated monomer which is usable for carrying out the second- or subsequent step reversed phase suspension polymerization, or can be added to the reaction mixture obtained by first- or subsequent steps after cooling.

The phosphorus-containing compound is not particularly limited. The phosphorus-containing compound includes, for example, phosphorus acid compounds such as phosphorous acid or normal salts of phosphorous acid such as disodium phosphite, dipotassium phosphite, and diammonium phosphite, and acidic salts of phosphorous acid such as sodium hydrogenphosphite, potassium hydrogenphosphite, and ammonium hydrogenphosphite; phosphoric acid compounds such as phosphoric acid or normal salts of phosphoric acid such as sodium phosphate, potassium phosphate, and ammonium phosphate, and acidic salts of phosphoric acid such as sodium dihydrogenphosphate, potassium dihydrogenphosphate, ammonium dihydrogenphosphate, disodium hydrogenphosphate, dipotassium hydrogenphosphate, and diammonium hydrogenphosphate; hypophosphorous acid compounds such as hypophosphorous acid or salts of hypophosphorous acid such as sodium hypophosphite, potassium hypophosphite, and ammonium hypophosphite; pyrophosphoric acid, tripolyphosphoric acid, polyphosphoric acid, and salts thereof; trimethyl phosphate, nitrilomethylene triphosphonic acid, and the like. Each of these phosphorus-containing compounds may be used alone or in a mixture of two or more kinds. In addition, as the phosphorus-containing compound, a hydrate of these compounds may be used. Among the phosphorus-containing compounds, the phosphorous acid compounds, the phosphoric acid compounds, and the hypophosphorous acid compounds are preferable, and disodium phosphite, sodium dihydrogenphosphate, and sodium hypophosphite are more preferable, from the viewpoint that an effect exhibited by its addition is high.

It is desired that the amount of the above-mentioned phosphorus-containing compound in the step of carrying out the polymerization reaction by adding the phosphorus-containing compound to the reaction mixture in the polymerization reaction of the second- or subsequent-step is from 0.00001 to 0.05 mol, preferably from 0.00005 to 0.02 mol, and more preferably from 0.0001 to 0.01 mol per 1 mol of the water-soluble ethylenically unsaturated monomer subjected to the polymerization reaction. When the amount of the phosphorus-containing compound is less than 0.00001 mol per 1 mol of the water-soluble ethylenically unsaturated monomer, the effect of adding the phosphorus-containing compound is less likely to be sufficiently obtained, and when the amount exceeds 0.05 mol, the polymerization rate is likely to be delayed, and the amount of water-soluble substance is likely to be large.

In the present invention, it is preferable that post-crosslinking treatment is carried out by reacting the resulting water-absorbent resin with a post-crosslinking agent having at least two functional groups having reactivity with carboxyl groups.

The post-crosslinking agent may be any one that is reactive with a carboxyl group of the water-absorbent resin. Representative examples of the post-crosslinking agent include diols, triols and polyols such as (poly)ethylene glycol, (poly)propylene glycol, 1,4-butanediol, trimethylolpropane, polyoxyethylene glycol, polyoxypropylene glycol and (poly)glycerol; diglycidyl ether compounds such as (poly)ethylene glycol diglycidyl ether, (poly)propylene glycol diglycidyl ether and (poly)glycerol diglycidyl ether; epihalohydrin compounds such as epichlorohydrin, epibromohydrin and $\alpha$-methylepichlorohydrin; compounds each having at least two reactive functional groups, such as isocyanate compounds such as 2,4-tolylene diisocyanate and hexamethylene diisocyanate; and the like. These post-crosslinking agents can be used alone or in a mixture of at least two kinds.

The amount of the post-crosslinking agent cannot be unconditionally determined because the amount differs depending upon the kinds of the crosslinking agents. Usually, it is desired that the amount of the post-crosslinking agent is from 0.00001 to 0.01 mol, preferably from 0.00005 to 0.005 mol, more preferably from 0.0001 to 0.002 mol, per 1 mol of the total amount of the water-soluble ethylenically unsaturated monomer used for the polymerization. When the amount of the post-crosslinking agent used is less than 0.00001 mol, the water-absorbent resin is less likely to achieve a satisfactorily high crosslink density, and when the amount exceeds 0.01 mol, the amount of the crosslinking agent becomes excessive, so that an unreacted crosslinking agent is likely to remain.

The timing for adding the post-crosslinking agent is not particularly limited, as long as the post-crosslinking agent is added after the termination of the polymerization reaction of the monomer.

It is preferable that the water-absorbent resin and the post-crosslinking agent are mixed together in the presence of water. The amount of water used in the mixing differs depending upon the kinds, particle sizes, and water content of the water-absorbent resin. Usually, it is desired that the amount of water is from 5 to 300 parts by weight, preferably from 10 to 100 parts by weight, more preferably from 10 to 50 parts by weight, based on 100 parts by weight of the total amount of the water-soluble ethylenically unsaturated monomer used for polymerization. When the amount of water is less than 5 parts by weight, based on 100 parts by weight of the total amount of the water-soluble ethylenically unsaturated monomer used for polymerization, the crosslinking reaction is less likely to progress, and when the amount exceeds 300 parts by weight, the drying time is extended, thereby making it likely to be economically disadvantageous. The amount of water in the present invention means the total amount of water remaining in the reaction system and water used as occasion demands when the post-crosslinking agent is added.

After the termination of the post-crosslinking, water and the hydrocarbon-based solvent are distilled off from the water-absorbent resin by distillation, whereby a dry product of the water-absorbent resin can be obtained.

EXAMPLES

The present invention will be specifically described hereinbelow by the Examples, without intending to limit the scope of the present invention thereto.

Example 1

The amount 340 g of n-heptane and 0.92 g of a sucrose fatty acid ester (manufactured by MITSUBISHI CHEMICAL CORPORATION under the trade name of S-370) were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to dissolve the mixture, and thereafter the resulting solution was cooled to 55°C.

Separately from the above, 92 g of an 80% by weight aqueous solution of acrylic acid (1.02 mol) was added to a 500 mL-Erlenmeyer flask. Thereto was added dropwise 102.2 g of a 30% by weight aqueous sodium hydroxide (0.77 mol) with cooling from external, to neutralize 75% by mol of acrylic acid. Further, 50.2 g of water, 0.11 g (0.00041 mol) of potassium persulfate, and 8.3 mg (0.000047 mol) of ethylene glycol diglycidyl ether were added thereto, to give an aqueous monomer solution for a first-step polymerization.

The entire amount of this aqueous monomer solution for a first-step polymerization was added to the above-mentioned round bottomed flask with stirring, and the mixture was dispersed. After the internal of the system was sufficiently replaced with nitrogen gas, the temperature of the content mixture was raised to 70°C, and the polymerization reaction was carried out for 1 hour while keeping its bath temperature at 70°C. Thereafter, the reaction mixture in the form of a slurry was cooled to room temperature.

Separately from the above, 119.1 g of an 80% by weight aqueous solution of acrylic acid (1.32 mol) was added to a separate 500 mL-Erlenmeyer flask. The amount 132.2 g of a 30% by weight aqueous sodium hydroxide (0.99 mol) was added dropwise thereto while cooling, to neutralize 75% by mol of acrylic acid. Further, 27.4 g of water, 0.14 g (0.00052 mol) of potassium persulfate and 0.54 g (0.0025 mol) of disodium phosphite pentahydrate were added thereto, to give an aqueous monomer solution for a second-step polymerization. The aqueous monomer solution was cooled in an ice water bath.

The entire amount of this aqueous monomer solution for a second-step polymerization was added to the reaction mixture obtained above. Thereafter, the internal of the system was again sufficiently replaced with nitrogen gas, the temperature of the liquid mixture was then raised to 70°C, and the second-step polymerization reaction was carried out for 2 hours while keeping its bath temperature at 70°C. After the termination of the polymerization, the polymerization slurry was heated in an oil bath at 120°C, and the heated mixture was subjected to azeotropic distillation to distill off 261 g of water to external of the system. The amount of remaining water in the reaction system at this point was 56 g.

Next, 7.81 g of a 2% by weight aqueous solution of ethylene glycol diglycidyl ether (0.00090 mol) was added to the flask while mixing, and a post-crosslinking treatment was carried out. Water and n-heptane were further distilled off from the mixture by distillation, and the residue was dried, to give 223.1 g of a water-absorbent resin having a mass-average particle size of 380 $\mu$m.

Example 2

The same procedures as in Example 1 were carried out except that the amount of disodium phosphite pentahydrate in Example 1 was changed to 0.36 g (0.0017 mol), to give 222.5 g of a water-absorbent resin having a mass-average particle size of 370 $\mu$m.

Example 3

The amount 340 g of n-heptane and 0.92 g of a sucrose fatty acid ester (manufactured by MITSUBISHI CHEMICAL CORPORATION under the trade name of S-370) were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to dissolve the mixture, and thereafter the resulting solution was cooled to 55°C.

Separately from the above, 92 g of an 80% by weight aqueous solution of acrylic acid (1.02 mol) was added to a 500 mL-Erlenmeyer flask. Thereto was added dropwise 102.2 g of a 30% by weight aqueous sodium hydroxide (0.77 mol) with cooling from external, to neutralize 75% by mol of acrylic acid. Further, 50.2 g of water, 0.11 g (0.00041 mol) of potassium persulfate, and 8.3 mg (0.000047 mol) of ethylene glycol diglycidyl ether were added thereto, to give an aqueous monomer solution for a first-step polymerization.

The entire amount of this aqueous monomer solution for a first-step polymerization was added to the above-mentioned round bottomed flask with stirring, and the mixture was dispersed. After the internal of the system was sufficiently replaced with nitrogen gas, the temperature of the liquid mixture was raised to 70°C, and the polymerization reaction was carried out for 1 hour while keeping its bath temperature at 70°C. Thereafter, the reaction mixture in the form of a slurry was cooled to room temperature.

Separately from the above, 119.1 g of an 80% by weight aqueous solution of acrylic acid (1.32 mol) was added to a 500 mL-Erlenmeyer flask. The amount 132.2 g of a 30% by weight aqueous sodium hydroxide (0.99 mol) was added dropwise thereto with cooling, to neutralize 75% by mol of acrylic acid. Further, 27.4 g of water, 0.14 g (0.00052 mol) of potassium persulfate and 0.77 g (0.0036 mol) of disodium phosphite pentahydrate were added thereto, to give an aqueous monomer solution for a second-step polymerization. The aqueous monomer solution was cooled in an ice water bath.

The entire amount of this aqueous monomer solution for a second-step polymerization was added to the above-mentioned reaction mixture in the form of a slurry. After the internal of the system was again sufficiently replaced with nitrogen gas, the temperature of the liquid mixture was raised, and the second-step polymerization reaction was carried out for 2 hours with keeping its bath temperature at 70°C. After the termination of the polymerization, the polymerization slurry was heated in an oil bath at 120°C, and the heated mixture was subjected to azeotropic distillation to distill off 257 g of water to external of the system. The amount of remaining water in the reaction system at this point was 61 g.

Next, 7.81 g of a 2% by weight aqueous solution of ethylene glycol diglycidyl ether (0.00090 mol) was added thereto while mixing, and a post-crosslinking treatment was carried out. Water and n-heptane were further distilled off from the mixture by distillation, and the residue was dried, to give 224.5 g of a water-absorbent resin having a mass-average particle size of 370 $\mu$m.

<u>Example 4</u>

The amount 340 g of n-heptane and 0.92 g of a sucrose fatty acid ester (manufactured by MITSUBISHI CHEMICAL CORPORATION under the trade name of S-370) were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to dissolve the mixture, and thereafter the resulting solution was cooled to 55°C.

Separately from the above, 92 g of an 80% by weight aqueous solution of acrylic acid (1.02 mol) was added to a 500 mL-Erlenmeyer flask. Thereto was added dropwise 102.2 g of a 30% by weight aqueous sodium hydroxide (0.76 mol) while cooling from external, to neutralize 75% by mol of acrylic acid. Further, 50.2 g of water, 0.11 g (0.00041 mol) of potassium persulfate, and 8.3 mg (0.000048 mol) of ethylene glycol diglycidyl ether were added thereto, to give an aqueous monomer solution for a first-step polymerization.

The entire amount of this aqueous monomer solution for a first-step polymerization was added to the above-mentioned five-necked cylindrical round bottomed flask with stirring, and the mixture was dispersed. After the internal of the system was sufficiently replaced with nitrogen gas, the temperature of the mixture was raised, and the polymerization reaction was carried out for 1 hour while keeping its bath temperature at 70°C. Thereafter, the reaction mixture in the form of a slurry was cooled to room temperature.

Separately from the above, 119.1 g of an 80% by weight aqueous solution of acrylic acid (1.32 mol) was added to a 500 mL-Erlenmeyer flask. The amount 132.2 g of a 30% by weight aqueous sodium hydroxide (0.99 mol) was added dropwise thereto while cooling, to neutralize 75% by mol of acrylic acid. Further, 27.4. g of water, 0.14 g (0.00052 mol) of potassium persulfate, 10.7 mg (0.000062 mol) of ethylene glycol diglycidyl ether, and 0.54 g (0.0025 mol) of disodium phosphite pentahydrate were added thereto, to give an aqueous monomer solution for a second-step polymerization. The aqueous monomer solution was cooled in an ice water bath.

The entire amount of this aqueous monomer solution for a second-step polymerization was added to the above-mentioned reaction mixture in the form of a slurry. After the internal of the system was again sufficiently replaced with nitrogen gas, the temperature of the mixture was raised, and the second-step polymerization reaction was carried out for 2 hours with keeping its bath temperature at 70°C. After the termination of the polymerization, the polymerization slurry was heated in an oil bath at 120°C, and the heated mixture was subjected to azeotropic distillation to distill off 263 g of water to external of the system. The amount of remaining water in the reaction system at this point was 54 g.

Next, 7.81 g of a 2% by weight aqueous solution of ethylene glycol diglycidyl ether (0.00090 mol) was added thereto while mixing, and a post-crosslinking treatment was carried out. Water and n-heptane were further distilled off from the mixture by distillation, and the residue was dried, to give 222.5 g of a water-absorbent resin having a mass-average particle size of 382 $\mu$m.

Example 5

The same procedures as in Example 1 were carried out except that 1.19 g (0.0076 mol) of sodium dihydrogenphosphate dihydrate was used in place of 0.54 g (0.0025 mol) of disodium phosphite pentahydrate in Example 1, to give 221.7 g of a water-absorbent resin having a mass-average particle size of 385 $\mu$m.

Example 6

The same procedures as in Example 1 were carried out except that 0.018 g (0.00017 mol) of sodium hypophosphite monohydrate was used in place of 0.54 g (0.0025 mol) of disodium phosphite pentahydrate in Example 1. to give 221.8 g of a water-absorbent resin having a mass-average particle size of 375 $\mu$m.

Comparative Example 1

The same procedures as in Example 1 were carried out except that disodium phosphite pentahydrate in Example 1 was not used, to give 220.9 g of a water-absorbent resin having a mass-average particle size of 380 $\mu$m.

Comparative Example 2

The amount 340 g of n-heptane and 0.92 g of a sucrose fatty acid ester (manufactured by MITSUBISHI CHEMICAL CORPORATION under the trade name of S-370) were added to a 1000 mL-five-necked cylindrical round bottomed flask equipped with a stirrer, a reflux condenser, a dropping funnel, a thermometer and a nitrogen gas inlet tube. The mixture was dispersed in the flask, and the temperature of the dispersion was raised to dissolve the mixture, and thereafter the resulting solution was cooled to 55°C.

Separately from the above, 92 g of an 80% by weight aqueous solution of acrylic acid (1.02 mol) was added to a 500 mL-Erlenmeyer flask. Thereto was added dropwise 102.2 g of a 30% by weight aqueous sodium hydroxide (0.76 mol) with cooling from external, to neutralize 75% by mol of acrylic acid. Further, 50.2 g of water, 0.11 g (0.00041 mol) of potassium persulfate, 8.3 mg (0.000048 mol) of ethylene glycol diglycidyl ether, and 0.41 g (0.0019 mol) of disodium phosphite pentahydrate were added thereto, to give an aqueous monomer solution for a first-step polymerization.

The entire amount of this aqueous monomer solution for a first-step polymerization was added to the above-mentioned five-necked cylindrical round bottomed flask with stirring, and the mixture was dispersed. After the internal of the system was sufficiently replaced with nitrogen gas, the temperature of the mixture was raised, and the polymerization reaction was carried out for 1 hour while keeping its bath temperature at 70°C. Thereafter, the reaction mixture in the form of a slurry was cooled to room temperature.

Separately from the above, 119.1 g (1.32 mol) of an 80% by weight aqueous solution of acrylic acid was added to a 500 mL-Erlenmeyer flask. The amount 132.2 g (0.99 mol) of a 30% by weight aqueous sodium hydroxide solution was added dropwise thereto while cooling, to neutralize 75% by mol of acrylic acid. Further, 27.4 g of water, 0.14 g (0.00052 mol) of potassium persulfate were added thereto, to give an aqueous monomer solution for a second-step polymerization. The aqueous monomer solution was cooled in an ice water bath.

The entire amount of this aqueous monomer solution for a second-step polymerization was added to the above-mentioned reaction mixture in the form of a slurry. After the internal of the system was again sufficiently replaced with nitrogen gas, the temperature of the mixture was raised, and the second-step polymerization reaction was carried out for 2 hours with keeping its bath temperature at 70°C. After the termination of the polymerization, the polymerization slurry was heated in an oil bath at 120°C, and the heated mixture was subjected to azeotropic distillation to distill off 257 g of water to external of the system. The amount of remaining water in the reaction system at this point was 61 g.

Next, 7.81 g of a 2% by weight aqueous solution of ethylene glycol diglycidyl ether (0.00090 mol) was added thereto while mixing, and a post-crosslinking treatment was carried out. Water and n-heptane were further distilled off from the mixture by distillation, and the residue was dried, to give 223.5 g of a water-absorbent resin having a mass-average particle size of 380 $\mu$m.

The water-absorbent resin obtained in each Example and each Comparative Example was evaluated in accordance with the following methods. The evaluation results for the following items (1) to (5) are shown in Table 1.

(1) Water-Retaining Capacity of Physiological Saline

Two grams of a water-absorbent resin was weighed in a cotton bag (Cottonbroad No. 60, width 100 mm x length 200 mm), and placed in a 500 mL-beaker. Physiological saline was poured into the cotton bag in an amount of 500 g at a time, and the saline was dispersed so as not to generate an unswollen lump of the water-absorbent resin. The upper part of the cotton bag was tied up with a rubber band, and the cotton bag was allowed to stand for 1 hour, to sufficiently swell the water-absorbent resin. The cotton bag was dehydrated for 1 minute with a dehydrator (manufactured by Kokusan Enshinki Co., Ltd., product number: H-122) set to have a centrifugal force of 167G, and the weight (Wa) (g) of the cotton bag containing swelled gels after the dehydration was determined. The same procedures were carried out without adding a water-absorbent resin, and the empty weight (Wb) (g) of the cotton bag upon wetting was determined. The water-retaining capacity of physiological saline was calculated from the following formula:

[Water-Retaining Capacity of Physiological Saline] (g/g)

= [Wa - Wb] (g)/ [Weight of Water-Absorbent Resin] (g) .

(2) Amount of Water Absorption of Physiological Saline under Pressure (1960 Pa)
The amount of water absorption of physiological saline of a water-absorbent resin under the pressure of 1960 Pa was determined using a measuring apparatus X shown in Figure 1.

The measuring apparatus X shown in Figure 1 comprises a balance 1, a bottle 2 placed on the balance 1, an air aspiration tube 3, a lead tube 4, a glass filter 5, and a measuring section 6 placed on the glass filter 5.

The balance 1 is connected to a computer 7 so that change in weight can be recorded in the units of seconds or minutes. The bottle 2 holds physiological saline in the internal thereof, and the air aspiration tube 3 is inserted in an opening on its top, and the lead tube 4 is attached to the body section. The lower end of the air aspiration tube 3 is soaked in the physiological saline 8. The glass filter 5 has a diameter of 25 mm. As the glass filter 5, Glass Filter No. 1 of NIPPON RIKAGAKU KIKAI CO., LTD. (pore size: 100 to 160 $\mu$m) was used.

The bottle 2 and the glass filter 5 are communicated with each other via the lead tube 4. In addition, the glass filter 5 is fixed to a position slightly higher than the lower end of the air aspiration tube 3. The measuring section 6 has a cylinder 60, a nylon mesh 61 adhered to the bottom part of the cylinder 60, and a weight 62 having a weight of 62.8 g. The cylinder 60 has an inner diameter of 20 mm. The nylon mesh 61 is formed to have a size of 200 mesh screen (size of opening: 75 $\mu$m), and a given amount of a water-absorbent resin 9 is evenly spread over the nylon mesh 61. The weight 62 is placed on the water-absorbent resin 9, so that a 1960 Pa load can be applied to the water-absorbent resin 9.

In the measuring apparatus X having the constitution as described above, first, the bottle 2 is charged with physiological saline in a given amount, and the air aspiration tube 3 is placed in the bottle 2 to get ready for the determination. Next, 0.1 g of the water-absorbent resin 9 is evenly spread over the nylon mesh 61 in the cylinder 60, and the weight 62 is placed on the water-absorbent resin 9. The measuring section 6 is placed on the glass filter 5 so that its central section is in alignment with the central section of the glass filter 5.

On the other hand, the computer 7 connected to the electronic balance 1 is booted, and the weight reduction of the physiological saline in the bottle 2, i.e., the weight of the physiological saline absorbed by the water-absorbent resin 9, Wj (g), is continuously recorded on the computer 7 from the time when the water-absorbent resin 9 starts absorbing water, in units of minutes and preferably in units of seconds on the basis of the value obtained from the balance 1. The amount of water absorption of physiological saline of the water-absorbent resin 9 under pressure after 60 minutes passed from the beginning of the water absorption is obtained by dividing the weight Wc (g) after 60 minutes passed by the weight of the water-absorbent resin 9 (0.1 g)

(3) Amount of Water Absorption of Physiological Saline under Pressure (3920 Pa)
The amount of water absorption of physiological saline of a water-absorbent resin under the pressure of 3920 Pa was determined in the same manner as in the above-mentioned (2), except that the weight 62 was changed from 62.8 g to 125.6 g in the determination method of the above-mentioned (2), to obtain the amount of water absorption of physiological saline under the pressure of 3920 Pa.

(4) Water Absorption Rate
The amount 50 $\pm$ 0.01 g of physiological saline at a temperature of 23° to 26°C was weighed out in a 100 mL beaker. A magnetic stirrer bar having a size of 8 mm x 30 mm without a ring was placed in the beaker, and the beaker was placed on the top of MAGNETIC STIRRER (manufactured by IUCHI under the product number of HS-30D). Subsequently, the magnetic stirrer bar was adjusted so that the magnetic stirrer bar rotated at 600 ppm, and further adjusted so that the bottom of the vortex generated by the rotation of the magnetic stirrer bar came near the upper portion of the magnetic stirrer bar.

Next, 1.0 $\pm$ 0.002 g of particles obtained by sieving the water-absorbent resins with two kinds of standard sieve complying with JIS-Z8801 (1982) (opening of sieve: 500 $\mu$m or 300 $\mu$m), and subjecting the sieved water-absorbent resins to a particle size adjustment (500 $\mu$m or less and 300 $\mu$m or more) were quickly poured between the center of vortex in the beaker and the side of the beaker, and the time (seconds) from a point where the water-absorbent resin was poured into the beaker to a point where the vortex converged was determined with a stopwatch, which is defined as a water absorption rate.

(5) Amount of Water-Soluble Substance
The amount 500 $\pm$ 0.1 g of physiological saline was weighed out in a 500 mL beaker. A magnetic stirrer bar having a size of 8 mm $\times$ 30 mm without a ring was placed in the beaker, and the beaker was placed on the top of MAGNETIC STIRRER (manufactured by IUCHI under the product number of HS-30D). Subsequently, the magnetic stirrer bar was adjusted so that the magnetic stirrer bar rotated at 600 ppm, and further adjusted so that the bottom of the vortex generated by the rotation of the magnetic stirrer bar came near the upper portion of the magnetic stirrer bar.

Next, 2.0 ± 0.002 g of particles obtained by sieving the water-absorbent resins with two kinds of standard sieve complying with JIS-Z8801-1982 (opening of sieve: 500 μm or 300 μm), and subjecting the sieved water-absorbent resins to a particle size adjustment (500 μm or less and 300 μm or more) were quickly poured between the center of vortex in the beaker and the side of the beaker and dispersed therein, and the mixture was stirred for 3 hours. The aqueous dispersion of the water-absorbent resin after stirring for 3 hours was filtered with a standard sieve (opening of sieve: 75 μm), and the filtrate obtained was further subjected to suction filtration using a Kiriyama type funnel (Filter Paper No. 6).

The amount 80 ± 0.1 g of the filtrate obtained was weighed out in a 100 mL beaker dried beforehand to a constant weight, and the filtrate was dried with a forced convection oven (manufactured by ADVANTEC) at 140°C until a constant weight is attained. A weight Wd (g) of the solid content of the filtrate was determined.

On the other hand, the same procedures as the above were carried out without using the water-absorbent resins, and a weight We (g) of the solid content of the filtrate was determined. The amount of water-soluble substance was calculated on the basis of the formula:

$$\text{Amount of Water-Soluble Substance (\% by weight)}$$

$$= [[(Wd - We) \times (500/80)]/2] \times 100 .$$

(6) Mass-Average Particle Size

One-hundred grams of a water-absorbent resin was weighed and placed on an uppermost sieve of 8 standard sieves complying with JIS-Z 8801(1982) (openings of sieves: 850 μm, 500 μm, 355 μm, 300 μm, 250 μm, 180 μm, 106 μm and the bottom of the container being stacked in order from the top), and shaken with a rotating and tapping shaker machine for 10 minutes to sieve the water-absorbent resin, and thereafter the water-absorbent resin remaining on each sieve was weighed. In accordance with the results, the particle size of which cumulative mass is 50% was calculated in accordance with the following formula:

$$[\text{Mass-Average Particle Size (μm)}] = [(50-a)/(d-a)] \times (c - b) + b$$

wherein a is the cumulative value (g) obtained when the mass is sequentially accumulated from those having coarser particle sizes in the distribution, to obtain a cumulative value of which cumulative mass is less than 50% by mass and most closely approximating 50% by mass; b is an opening (μm) of the sieve at which the cumulative value is obtained; d is the accumulated value obtained when the mass is sequentially accumulated from those having coarse particle sizes in the distribution to obtain a cumulative value of which cumulative mass is at least 50% by mass and most closely approximating 50% by mass; and c is an opening (μm) of the sieve at which the cumulative value is obtained.

[Table 1]

| Ex. No. and Comp. Ex. No. | Water-Retaining Capacity of Physiological Saline (g/g) | Amount of Water Under Pressure (g/g) | | Absorption | Water Absorption Rate (sec) | Amount of Water-Soluble Substance (% by wt.) |
|---|---|---|---|---|---|---|
| | | 1960 Pa | 3920 Pa | | | |
| Ex. 1 | 47 | 37 | 23 | 41 | 16 |
| Ex. 2 | 44 | 38 | 25 | 38 | 15 |
| Ex. 3 | 48 | 37 | 22 | 36 | 20 |
| Ex. 4 | 43 | 37 | 24 | 39 | 17 |
| Ex. 5 | 46 | 36 | 23 | 41 | 18 |
| Ex. 6 | 47 | 38 | 22 | 39 | 19 |
| Comp. Ex. 1 | 39 | 31 | 17 | 47 | 21 |
| Comp. Ex. 2 | 46 | 36 | 23 | 70 | 35 |

It can be seen from the results shown in Table 1 that the water-absorbent resin obtained in each Example has a large water-retaining capacity of physiological saline, a large amount of water absorption of physiological saline under pressure, a high water absorption rate, and a small amount of water-soluble substance.

INDUSTRIAL APPLICABILITY

The water-absorbent resin obtained by the process for preparing a water-absorbent resin of the present invention can be suitably used in hygienic materials such as disposable diaper, incontinence pad and sanitary napkin, especially in disposable diaper.

**Claims**

1. A process for preparing a water-absorbent resin comprising carrying out a reversed phase suspension polymerization in multi-steps of at least two steps when the water-absorbent resin is prepared by subjecting a water-soluble ethylenically unsaturated monomer to the reversed phase suspension polymerization, said process for preparing a water-absorbent resin being **characterized by** adding a phosphorus-containing compound to at least one step in the second and subsequent steps, to carry out the polymerization reaction.

2. The process for preparing a water-absorbent resin according to claim 1, wherein the phosphorus-containing compound is at least one member selected from the group consisting of phosphorous acid compounds, phosphoric acid compounds and hypophosphorous acid compounds.

3. The process for preparing a water-absorbent resin according to claim 1 or 2, wherein the amount of the phosphorus-containing compound is from 0.00001 to 0.05 mol per 1 mol of the water-soluble ethylenically unsaturated monomer used in the polymerization reaction in the step of adding a phosphorus-containing compound, to carry out polymerization reaction.

[Figure 1]

## INTERNATIONAL SEARCH REPORT

| International application No. |
| --- |
| PCT/JP2004/019022 |

**A. CLASSIFICATION OF SUBJECT MATTER**
Int.Cl$^7$ C08F2/20, 2/38

According to International Patent Classification (IPC) or to both national classification and IPC

**B. FIELDS SEARCHED**

Minimum documentation searched (classification system followed by classification symbols)
Int.Cl$^7$ C08F2/00-2/60

Documentation searched other than minimum documentation to the extent that such documents are included in the fields searched

Electronic data base consulted during the international search (name of data base and, where practicable, search terms used)
WPI/L

**C. DOCUMENTS CONSIDERED TO BE RELEVANT**

| Category* | Citation of document, with indication, where appropriate, of the relevant passages | Relevant to claim No. |
| --- | --- | --- |
| X | JP 9-12613 A (Mitsubishi Chemical Corp.), 14 January, 1997 (14.01.97), Claims; examples 1 to 15 & EP 751159 B & US 5652309 A & DE 69610039 T2 & CN 1146997 A | 1-3 |
| X | JP 11-130968 A (Mitsubishi Chemical Corp.), 18 May, 1999 (18.05.99), Claims; examples 1 to 9 (Family: none) | 1-3 |
| A | JP 9-143210 A (Sumitomo Seika Chemicals Co., Ltd.), 03 June, 1997 (03.06.97), Claims (Family: none) | 1-3 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 January, 2005 (27.01.05) | 15 February, 2005 (15.02.05) |

| Name and mailing address of the ISA/ Japanese Patent Office | Authorized officer |
| --- | --- |
| Facsimile No. | Telephone No. |

Form PCT/ISA/210 (second sheet) (January 2004)

**INTERNATIONAL SEARCH REPORT**

| International application No. |
|---|
| PCT/JP2004/019022 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 7-116511 A (Elf Atochem S.A.), 09 May, 1995 (09.05.95), Claims & EP 644211 B & FR 2710342 A & US 5563218 A & TW 300229 A & CN 1102832 A & DE 69405839 T2 & SG 52726 A | 1-3 |
| A | JP 5-17509 A (Mitsubishi Petrochemical Co., Ltd.), 26 January, 1993 (26.01.93), Claims & EP 522570 B & US 5548047 A & TW 242152 A & DE 69219223 T2 & KR 204464 B | 1-3 |
| A | JP 2-255804 A (Nippon Shokubai Kagaku Kogyo Co., Ltd.), 16 October, 1990 (16.10.90), Claims & EP 372981 B & US 6335406 B | 1-3 |

Form PCT/ISA/210 (continuation of second sheet) (January 2004)

**EP 1 714 985 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6345819 A
- JP 3227301 A
- JP 8120013 A
- JP 6287233 A
- JP 9124710 A